# EUROPEAN PATENT SPECIFICATION

(11) **EP 0 578 754 B1**
(45) Date of publication and mention of the grant of the patent: **07.06.1995**
(21) Application number: 92910290.3
(22) Date of filing: 27.03.1992
(51) Int. Cl.: C12N 15/71, C12N 15/16, C12N 1/21

(54) **TrpR MUTATIONS EFFECTIVE IN THE EXPRESSION OF SMALL PROTEINS AND PEPTIDES**
TrpR MUTATIONEN DIE WIRKSAM SIND IN DER EXPRESSION VON KLEINEN PROTEINEN UND PEPTIDEN
MUTATIONS DE TrpR SERVANT A L'EXPRESSION DE PETITES PROTEINES ET DE PETITS PEPTIDES

(30) Priority: 05.04.1991 US 681688; 18.02.1992 US 837313
(43) Date of publication of application: 19.01.1994
(73) Proprietor: THE UPJOHN COMPANY, Kalamazoo, Michigan 49001 (US)
(72) Inventor: LEPLEY, Robert, Alan, Plainwell, MI 49080 (US)
(74) Representative: Perry, Robert Edward
(86) International application number: US9202235
(87) International publication number: WO9217594

(56) References cited:
- EP-A- 0 155 655
- EP-A- 0 197 797
- METHODS IN ENZYMOLOGY, vol. 101, 1983, pp. 155-164; B. NICHOLS AND C. YANOFSKY;
- PROCEEDINGS OF THE NATIONAL ACADEMY OF SCIENCES OF USA. vol. 63, 1969, WASHINGTON US pages 392 - 399; L. SOLL AND P. BERG: 'Recessive lethals: a new class of nonsense suppressors in Escherichia coli' cited in the application
- JOURNAL OF BIOLOGICAL CHEMISTRY. vol. 265, no. 2, 15 January 1990, BALTIMORE US pages 731 - 737; J-J. HE AND K MATTHEWS: 'Effect of amino acid alterations in the tryptophan-binding site of the Trp-repressor'
- H. SCHLEGEL 'Allgemeine Mikrobiologie' 1972 , GEORG THIEME VERLAG , STUTTGART

## Description

### Field of the Invention

This invention is in the fields of recombinant genetic engineering and heterologous protein expression and provides novel strains of *E. coli* exhibiting enhanced protein expression. This invention also provides a novel method of creating organisms exhibiting enhanced protein expression.

### Background of the Invention

The monomeric expression of small proteins and peptides in microorganisms has proved difficult to accomplish on a scale which yields appreciable quantities of protein. The basis for this is not understood, but has been attributed to a lack of product stability rather than transcriptional or translational concerns. Therefore, attempts have been made to increase product stability through the use of protease deficient hosts, by directing product into the periplasmic space to escape intra-cellular proteases, by increasing product size through the use of gene fusions or chimeric genes, or through the use of co-expressed chaperon proteins.

A somewhat different approach involves enhancing protein synthesis by increasing the intracellular concentrations of aminoacyl-transfer RNAs (aa-tRNA) and thereby increasing the rate of chain elongation. However, this technique has met with little success *in vivo*.

The method described here modifies or alters the regulation of the tryptophan operon as a means of increasing protein expression. The tryptophan operon, which maps at minute 28 of the *E. coli* chromosome, encodes structural genes for the various proteins necessary for the biosynthesis of L-tryptophan, an essential amino acid. The operon is tightly regulated through a system of both repression and attenuation at the level of transcription, and, in addition, is regulated via translational controls. (For a complete discussion of regulation of the tryptophan operon, see C. Yanofsky and I. P. Crawford (1987), *The Tryptophan Operon*, F.C. Niedhardt (Ed.), *Escherichia coli and Salmonella typhimurium*, vol. II, chapter 90, pp. 1453-1472.)

One protein involved in the repression of the tryptophan operon is the tryptophan repressor protein (TrpR). The structural gene for TrpR encodes a 108-residue polypeptide which maps at minute 99 of the *E. coli* chromosome. In the presence of intracellular L-tryptophan concentrations exceeding TrpR binding requirements for the amino acid, TrpR combines with L-tryptophan to form a holoenzyme complex which has enhanced affinity for specific DNA sequences found within the tryptophan (trp) promoter. When the TrpR/trp holoenzyme complex binds to these trp promoter sequences, transcription of the entire trp operon is reduced to extremely low levels. Since the operon directs the transcription of proteins necessary for the biosynthesis of L-tryptophan, and due in part to concurrent intracellular proteolytic degradation of the biosynthetic enzymes, further production of L-tryptophan ceases and the level of intracellular L-tryptophan will be reduced. As the excess of L-tryptophan is depleted, the affinity of TrpR-trp complex for the operon is reduced allowing transcription rates to again increase. Ultimately, then, an excess of intracellular L-tryptophan serves to "turn off" synthesis of further L-tryptophan.

Alleles are known which encode variants of TrpR. The strains which carry these alleles display a whole range of functional effects, from TrpR phenotypes having very little effect on repressor binding to those which synthesize TrpR wholly unable to bind to the trp promoter. One such *tprR* mutant i*s trpR₅₅*. *trpR₅₅* has not been fully characterized but has been shown to fully "de-repress" the tryptophan operon. *See* Soll, L. and P. Berg (1969), Recessive lethals: A new class of nonsense supressors in Escherichia coli, *PNAS* (USA) 63:392-399. This allele may be translocated from one microorganism to another by the use of phages.

Phage transduction represents a convenient means for inserting genetic information into the chromosomal DNA of a microorganism and is used here to insert a nonfunctioning repressor gene into the genome of a host microorganism. While not wishing to be bound by any one theory, it is believed that the constitutive de-repression of the tryptophan operon that results from the action of this protein allows L-tryptophan to reach intracellular concentrations which exceed that found in wild-type cells. When the host is transformed with a recombinant plasmid encoding expression vectors for heterologous protein of economic or research interest, the derepressed plasmid promoter directs synthesis of the heterologous protein at a high rate. The protein is expressed as a monomeric product and, therefore, this invention eliminates the need for fusion peptides, chimeric genes, etc., and the additional purification steps which those expression methods employ. In addition, we have found that the monomeric protein product displays enhanced stability as compared to wild-type controls.

The invention described here uses phage technology to create microorganisms having a mutant *trpR* phenotype exhibiting reduced or absent operon control by TrpR (i.e. organisms which are phenotypically *trpR*⁻). This invention, then, provides new strains of *E. coli* capable of high level heterologous protein expression. Advantageously, the *E. coli* of the invention express protein as monomeric proteins or peptides, and, in addition, the expressed protein or peptide has a surprisingly long half-life. This invention also provides a novel method by which microorganisms may be genetically modified to enhance the monomeric expression of small heterologous proteins and peptides.

### Information Disclosure

Rojiani, M. V., et al (1990), "Relationship between protein synthesis and concentration of charged and uncharged tRNA^{Trp} in Escherichia", PNAS(USA) 87:1511-1515, report that the rate of incorporation of tryptophan into protein is proportional to the fraction of total tRNA^{Trp} which is Trp-tRNA^{Trp}. However, the report does not describe any alteration to the tryptophan repressor.

Soll et al (1969), PNAS(USA) 63:392-399, describe the discovery of new non-sense suppressors in *E. coli.* These suppressors are characterised using an *E. coli* strain which is phenotypically trpR⁻. This reference describes an *E. coli* strain subsequently placed on deposit at the *E. coli* Genetic Stock Center (CGSC) under the accession code LS340.

Yanofsky et al (1966), J. Mol. Biol. 21:313-334, describe studies done to verify polarity in the tryptophan operon and use *E. coli* which are phenotypically trpR⁻.

Olsen et al (1989), J. Biotechnology 9:179-190, describe the construction of the Trp2 vector, employed in plasmid pIGF70/Trp2.

### Summary of the Invention

In one aspect of this invention, host microorganisms have a chromosomally-inserted non-functional TrpR55 gene, and are *E. coli* strains LMS-73, LMS-117 and LMS-118. These strains may be transformed to encode heterologous protein. The preferred heterologous protein is IGF-I. The preferred vector for transformation into LMS-73 is pIGF-I. The preferred vector for transformation into LMS-117 and LMS-118 is pRAL1, a novel plasmid which encodes the P_{L} promoter and ribosomal binding site. In addition, pRAL1 contains the Trp2 ribosomal binding site upstream of a cDNA coding for IGF 70.

In another aspect of the invention, a host of the invention that comprises a vector encoding a heterologous protein, operably linked to a tryptophan promoter, is cultured, and the protein isolated. This method provides enhanced expression and stability of the protein.

### Detailed Description of the Invention

The method described here utilizes modifications or alterations to the tryptophan repressor gene (*trpR*) as a means of increasing protein expression and the stability thereof. Enhanced protein expression is accomplished by altering the chromosomal gene encoding trpR to create a microorganism having a phenotype displaying reduced, or absent, repression of the tryptophan operon, i.e. organisms which are phenotypically *trpR*⁻. Various methods are known in the art which may be employed to create a trpR⁻ microorganism. Common among these are chemical mutagenesis, UV mutagenesis, and phage transduction. Phage transduction is preferred as it represents a convenient means for insertion of the nonfunctional repressor gene.

The invention, described briefly here and in more detail below, is exemplified by insertion of a mutant *trpR* allele into the chromosomal DNA of *E. coli*. The insertion of the defective *trpR* allele is accomplished by the use of a transducing phage. A transducing phage has the unique ability to insert or remove chromosomal DNA into, or out of, a bacterial host.

The present invention is best exemplified by P1 phage transduction of *E. coli* strains NK5148 and LS340 into *E. coli* DU99b. P1 is obtained from the Upjohn Culture Collection. However, any P1 phage is suitable to perform the manipulations described here and a variety of P1 bacteriophages are available from commercial sources, e.g. American Type Culture Collection. NK5148 carries a gene encoding tetracycline resistance; LS340 carries a mutant *trpR* allele capable of generating a defective tryptophan repressor protein (TrpR) in *E. coli.* DU99b is transduced first with a phage generated from NK5148 and subsequently with a phage generated from LS340. In addition to LS340, various organisms are available which carry, or in the alternative may be engineered by one skilled in the art to carry, a chromosomal gene encoding nonfunctional tryptophan repressor protein and thus are also useful in the generation of *trpR*⁻ mutants. LS340 carries *trpR₅₅* and is available from the *E. coli* Genetic Stock Center (CGSC). Strains known to be *trpR*⁻ which are available from American Type Culture Collection (ATCC) include: LE392 which carries *trpR₅₅* (ATCC #33572); CY15070 carrying *trpR₂₁* (#47022); and JF242 carrying *trpR₅₅* (#47034).

*E. coli* DU99b (available from Northern Regional Research Laboratory (NRRL), U.S. Department of Agricultural, Peoria, IL, accession number B-18590) is the parent strain from which the new *E. coli* strains described here are derived. Organisms other than DU99b, of course, may be transduced following the method of the invention to produce other new strains capable of high level protein expression. For example, BST-1c, RV308, DH1, JM101, JM103, W3110, and K12, among others, which are known and commercially available (see e.g., American Type Culture Collection, "Catalog of Bacteria and Bacteriophages") may be used to practice the invention described here. In addition, the present invention is not limited to procaryotic organisms. Other organisms, such as yeast and it's various cloning vectors (e.g. YIP5), may also be employed as expression hosts.

Following the procedure described here, DU99b is modified via P1 phage transduction to produce a novel *E. coli* strain, LMS-73, which is deposited under the terms of the Budapest Treaty at the NRRL on 21 December 1990, and granted accession number NRRL B-18744.

LMS-73 is transformed with plasmid pIGF70/Trp2 for the enhanced expression of monomeric, biologically active, stabile, insulin-like growth factor-I (IGF-I). pIGF70/Trp2 may be isolated from *E. coli* RAL-4 following conventional means for plasmid isolation. RAL-4 is available from the NRRL, accession number B-18562. pIGF70/Trp2 codes for a 70 amino acid form of the IGF-I protein (IGF 70) linked downstream of a DNA sequence (Trp2) similar to that known to encode the *E. coli* tryptophan promoter and ribosomal binding site. IGF 70 is believed to be the form of IGF-I responsible for observed biological effects. IGF-I is a single polypeptide chain having a molecular weight of about 7,647 daltons. In eucaryotic cells naturally occurring IGF-I is translated from a primary mRNA transcript to a pre,pro-IGF-I protein from which a signal sequence of variable length and a C-terminal extension of 35 amino acids must be proteolytically cleaved to obtain the active form having 70 amino acids.

Alternatively, pIGF70/Trp2 may be constructed as briefly outlined below. First, a gene coding for insulin-like growth factor-I (IGF-I) is isolated from a porcine hepatic cDNA library, or in the alternative, a cDNA is synthesized using published amino acid sequences for porcine IGF. IGF-I expression vectors are then constructed by enzyme restriction and recombinant engineering of the cDNA by addition of linkers containing restriction sites designed to facilitate expression of the vector assembly, and initiation and N-terminal codons. Any appropriate restriction site may be engineered into the linker; the sites used in isolation of pIGF70/Trp2 are *ClaI* (C-terminus) and *BamHI* (N-terminus). Selection of an appropriate N-terminal codon, as well as the vector used in subsequent transformations, is also within the ordinary skill of one in the art. The isolated expression vector, which contains the 105 amino acid form of IGF-I (pIGF105) is ligated into a pBr-based vector, pSK4, which contains the *E. coli* tryptophan promoter and ribosomal binding site. This plasmid is designated pIGF105/Ptrp. pIGF105/Ptrp is then restricted, by use of *EcoRI* and *BglI*, and a fragment containing the 70 amino acid coding sequence for IGF-I and the tryptophan promoter isolated. This fragment is then ligated into pSK4, and is designated pIGF70/Ptrp. Restriction of pIGF70/Ptrp in turn provides a *ClaI/BamHI* fragment (which is IGF 70), which is isolated and ligated into a vector encoding the Trp promoter (Trp2). The construction of Trp2 is described by M.K. Olsen, et al, (1989), "Enhancement of Heterologous Polypeptide Expression by Alteration in the Ribosome-Binding Site Sequence", *J. Biotechnology* 9:179-190, which is incorporated by reference.

While pIGF70/Trp2 is a preferred vector for transforming LMS-73, any plasmid containing, or modified to contain, an expression vector encoding a suitable protein is useful to transform the novel organisms of the invention. For example, vectors suitable for expression of IGF-I in various pre-, pro-, and bioactive forms, include, pIGF70/Trp2, pIGF70/Trp4, pIGF105/Trp2, pIGF105/Trp4, pIGF70/ConSD, pIGF105/ConSD, pIGF70/TrpLE, and pIGF105/TrpLE. These expression vectors may be constructed by isolating the appropriate IGF-I (by utilizing the procedure outlined above) followed by insertion into the Trp2 vector, the ConSD vector as described by K.A. Curry and C-S.C. Tomich (1988), "Effect of ribosome binding site on gene expression in Escherichia coli", *DNA* 7:173-179, and the TrpLE vector as described by C-S. C. Tomich, et al (1988), "Use of *lacZ* Expression to Monitor Expression", *Plasmid* 20:167-170, which are incorporated by reference. In addition, to those skilled in the art it is apparent that any tryptophan promoted expression vector retaining the general properties of *trpR* regulation would be expected to function especially well as vectors in transforming the *trpR*⁻ mutants of the invention.

The manipulations and transformations described above will result in a microorganism which exhibits enhanced expression of the heterologous protein IGF-I. However, the protein expressed by the use of the method and constructs described here is not limited to IGF-I. Any low molecular weight polypeptide or protein having from 2 to about 150 amino acids is suitable for expression in the present invention. Examples of low molecular weight polypeptides and proteins include, insulin-like growth factor II (IGF-II), the A and B chains of human insulin, long chain somatotropin release inhibiting factor (somatostatin, SRIF), corticotrophin releasing factor (CRF), and proteins of the interleukin type (IL-I, IL-II, etc.), as well as functional analogs thereof. The term "low molecular weight polypeptides and proteins" also includes those proteins and peptides which are specific sub-domains of larger proteins which may be useful in the investigation of their function. The use of known low molecular weight proteins (e.g. IGF-I, GRF, IGF-II, SRIF, IL-I, IL-II, etc.) is well known in the art. Those skilled in the art will recognize other low molecular weight proteins which may be expressed and whose stability may be enhanced, by the techniques of the present invention. It should be apparent that the invention described here is useful both as a means of increasing expression of protein monomer and the stability thereof in the novel *E. coli* of the invention, in combination with known, or readily constructed, expression vectors, and also represents a general means of expression for proteins and peptides.

In addition, any plasmid containing, or modified to contain, the appropriate combination of regulatory element and protein gene may be used as a vector for the protein to be expressed. Various plasmids are known and easily modified to code for a desired protein. Appropriate plasmids include pBR322 and various derivatives, pUC19 and various derivatives, and various vectors known to run-away to extremely high copy numbers. Additional examples are set forth in Sambrook, J., et al (1989), *Molecular Cloning, A Laboratory Manual*, Cold Spring Harbor Laboratory Press, which is incorporated by reference.

In another aspect of the invention, LMS-73 is further modified by phage transduction to introduce into the bacterial host genome a marker gene coding for kanamycin (kan) marker linked to a sequence coding for cI⁸⁵⁷. Kanamycin is an antibiotic and the presence of the kanamycin gene renders the host kanamycin resistant. Thus, genomic incorporation of this marker allows for selection in the presence of the antibiotic.

*cI⁸⁵⁷* is a temperature-sensitive mutation to the bacteriophage λ repressor protein, cI. The *cI⁸⁵⁷* mutation encodes a gene product which is functional as cI repressor at temperatures below 32° C but undergoes thermal denaturation at temperatures in excess of 32 °C. The cI⁸⁵⁷ repressor is fully denatured and rendered nonfunctional at a temperature of about 42 °C and, therefore, expression experiments are conducted at about this temperature. The specific *cI⁸⁵⁷* allele used in the invention originated in *E. coli* strain TAP106 (*attN::kan*, *cI⁸⁵⁷*) and is a gift of D. Court (National Cancer Institute-Frederick Cancer Research and Development Center, Frederick, MD). *cI⁸⁵⁷* is transduced into the LMS-73 background using standard P1 phage methodology (Miller, 1972).

It is readily apparent that other suitable markers may also be incorporated into a host genome following the description given here. A variety of markers are known and the suitability of a particular marker, e.g. a marker which exhibits a particular color or antibiotic resistance, or a combination thereof, is known, or readily acquired, by one skilled in the art.

LMS-73 having the *kan-cI⁸⁵⁷* gene sequence is designated LMS-117 and is deposited under the terms of the Budapest Treaty at the Northern Regional Research Laboratory (NRRL) in Peoria, IL on 19 February 1991, and granted accession number NRRL B-18763.

LMS-73 is further modified by phage transduction to introduce a defective *lon* gene into the bacterial host genome. The *lon* gene codes for an ATP-activated intracellular protease which degrades small proteins and peptides as well as unstable proteins. The source of this gene is strain DU175C(106).1L (*lon::Tn10*/Tet) from the Upjohn Culture Collection. Any strain of *lon*⁻ *E. coli* may serve as a source of the defective *lon* gene. Numerous such strains are known, see e.g. Gottesman, S. (1990), "Minimaing Proteolysis in Escherichia coli: Genetic Solutions", *Methods in Enzymology* 185: 119-129, and Skorupski, K., et al (1988), "A Bacteriophage T4 Gene Which Functions To Inhibit Escherichia coli Lon Protease", *J. Bacteriol*. 170: 3016-3024.

LMS-73 having the *lon* gene sequence is designated LMS-79 and is deposited under the terms of the Budapest Treaty at the Northern Regional Research Laboratory (NRRL) in Peoria, IL on 20 March 1992, and granted accession number NRRL B-18951.

LMS-73 is again further modified by phage transduction to introduce a defective *lon* gene as well as the kanamycin marker-*cI⁸⁵⁷* sequence into the bacterial host genome.

This *lon-kan-cI⁸⁵⁷* strain is designated LMS-118 and deposited under the terms of the Budapest Treaty at the Northern Regional Research Laboratory (NRRL) in Peoria, IL on 19 February 1991, and granted accession number NRRL B-18764.

A further exemplification of the invention is in the construction of plasmid pRAL1 useful in the transformation of both LMS-117 and LMS-118 for the expression of IGF-I. In addition to the P_{L} promoter and ribosomal binding site this IGF expression vector is unique in that it also encodes Trp2 as well as the porcine IGF-I (active form) cDNA. This plasmid is constructed from a 7kb sequence, derived from pWS50, containing the P_{L} promoter and ribosomal binding site, linked to a sequence of pIGF70/Trp2 containing the Trp2 rbs and IGF-I 70 cDNA. pRAL1 is the preferred vector for transformation into LMS-117 and LMS-118.

pRAL1 is deposited under the terms of the Budapest Treaty at the Northern Regional Research Laboratory (NRRL), U.S. Department of Agricultural, Peoria, IL on 1 March 1991, and granted accession number NRRL B-18779.

Other vectors employing the stringently regulated bacteriophage λ promoter, P_{L}, are equally suitable for transformation into the novel organisms of the invention and are well known to those skilled in the art.

The following terms, as used throughout this application and claims, are defined as follows:
"Heterologous protein" refers to a protein which is encoded by a gene derived from an organism other than the host organism into which the gene is inserted.

"Enhanced production" or "enhanced expression" means a level of protein expression which is in excess of the amount expressed by wild-type controls.

"Increased stability" or "enhanced stability" means an increase in protein stability, as measured by the half-life of pulse-chase labelled heterologous protein, as compared to wild-type controls.

"Low molecular weight" or "LMW" refers to polypeptide containing from 2 to about 150 amino acids.

"OD₅₅₀" refers to the optical density of a solution as measured at a wavelength of 550 nanometers (nm).

Chart A illustrates the construction of pRAL1 of the present invention. The following conventions are used to illustrate plasmids and DNA fragments:
(1) The single line figures represent both circular and linear double-stranded DNA.
(2) Asterisks (*) indicate that the molecule represented is circular. Lack of an asterisk indicates the molecule is linear.
(3) Endonuclease restriction sites are indicated above the line.
(4) Genes are indicated below the line.
(5) Distance between genes and restriction sites are not to scale. The charts show their respective positions only.

Many of the methods employed to produce the novel organisms of the invention and the various embodiments thereof involve standard laboratory techniques of recombinant DNA engineering and phage manipulations. Such techniques are known by those skilled in the art or readily obtained by reference to standard laboratory texts. Examples of such texts are: J. Miller, *Experiments in Molecular Genetics*, Cold Spring Harbor Laboratory, Cold Spring Harbor, N.Y. (1972); D. A. Morrison, "Transformation and Preservation of Competent Bacterial Cells by Freezing", *Methods Enzymol*. 68:326-331 (1979); and Sambrook, J., et al (1989), *Molecular Cloning, A Laboratory Manual*, Cold Spring Harbor Laboratory Press, all of which are incorporated by reference. Except where noted, all chemicals and materials employed, or their equivalents, are readily available from commercial vendors.

The examples which follow serve to detail embodiments of the invention but in no way should be construed as a limitation of the invention.

### Material and Methods

Liquid and agar based media used in the maintenance of bacterial strains is purchased from DIFCO, Detroit, Michigan. All buffer components and wet chemistry agents are of reagent grade or better. Tetracycline, ampicillin, and kanamycin are obtained from Sigma, St. Louis, Missouri. Human recombinant IGF-I is used as an analytical standard and is available from IMCERA, Terre Haute, Indiana.

A wild-type *E. coli* phage P1 (The Upjohn Culture Collection) grown in K37 is used to generate P1 lysates on the appropriate donors. Transductions are performed as described by J. Miller, *Experiments in Molecular Genetics* (1972) and, where indicated, made use of strains obtained from B. Bachmann at the *E. coli* Genetic Stock Center (CGSC), Yale University, New Haven, Connecticut. Transformations are performed as described by D. A. Morrison, *Methods Enzymol*. 68:326-331 (1979).

To assess porcine IGF-I expression, *E. coli* are induced by growing overnight in LB/0.1% glucose supplemented with 100 »g/ml ampicillin, and 100 »g/ml L-tryptophan for pTrp vectors, and then diluting this saturated culture 1:100 into M9/0.2% glucose/1 % CAA/amp for induction. Samples are recovered from induced cultures and processed for sodium dodecyl sulfate polyacrylamide gel electrophoresis (SDS PAGE) and subsequent Western analysis.

SDS PAGE is performed using the method of U. K. Laemmli, "Cleavage of Structural Proteins During the Assembly of the Head of Bacteriophage T4", *Nature* 227:680-685 (1970). The discontinuous gel system found to best resolve IGF-I from *E. coli* proteins consists of a 15% separating gel and a 4% stacking gel. Immunological detection of IGF-I is carried out using rabbit polyclonal antiserum generated against human IGF-I. Western blots are executed essentially as described by Towbin et al., "Electrophoretic Transfer of Proteins from Polyacrylamide Gels to Nitrocellulose Sheets: Procedure and Some Applications", *PNAS(USA)* 76:4350-4354 (1979). In all induction experiments relative IGF-I expression is assessed qualitatively by Western analysis against 250 ng of IGF-I standard. Selection of this quantity of standard is based on appropriateness of autoradiographic signal strength generated by this quantity of standard relative to that expected from 0.25 OD₅₅₀/lane of a culture containing 1 »g IGF-I per unit OD₅₅₀.

### Example 1 IGF Expression by LMS-73

### a) Generation of Tryptophan Repressor Mutant (trpR⁻) LMS-73

*E. coli* strains LS340 (CGSC 5380; trpA9605, his-85, metE70, trpR55, λ⁻ , IN(rrnD-rrnE)1) and NK5148 (CGSC 6166; *thr₃₄::Tn10*, λ⁻, *IN(rrnD-rrnE)1*) are grown to saturation in LB/glucose. The media for NK5148 also contains 15 »g/ml tetracycline (Tet).

*E. coli* DU99b is transduced with phage P1^{(NK5148)} and the transductants which are *thr₃₄::Tn10* are selected on ABII/Tet plates. 50 colonies are picked onto M9/glucose/Tet and ABII/Tet. Four clones, phenotypically (Tet^{r}, thr⁻), are selected and grown to saturation in LB/glucose/Tet. These four clones are transduced with phage P1^{(LS340)} and plated on M9/glucose for thr⁺ selection. 25 clones are selected from each of the starting clones (100 clones total) and picked onto M9/glucose for purification. Of the 100 purified colonies, five from each of the starting clones (20 colonies total) are chosen for 5-methyltryptophan selection.

The colonies are streaked onto M9/glucose and M9/glucose supplemented with 100 »g/ml 5-methyltryptophan. Wild-type DU99b serve as controls. The plates are incubated at 37°C for 24 hours. Those clones successfully transduced to *trpR*⁻ via the P1^{(LS340)} transducing phage grow effectively in the presence of 5-methyltryptophan selection media while *trpR*⁺ transductants as well as *trpR*⁺ controls do not. Two of the 5-methyltryptophan resistant clones are selected and grown to saturation in LB/glucose.

The saturated cultures are streaked onto solid media to verify antibiotic resistance and phototropic growth. Both clones are verified and one is designated LMS-73 for subsequent use.

### b) IGF Expression

LMS-73 and DU99b are transformed with plasmid pIGF70/Trp2. This is a pBR322 based vector which codes for a promoter derived from the tryptophan operon linked to a nucleotide sequence for the 70 amino acid, physiologically-active, form of porcine IGF-I. The IGF 70 sequence is described above. The construction of the Trp2 vector deployed as the basis for construction of pIGF70/Trp2 has been described by M.K. Olsen, et al, (1989), *J. Biotechnology* 9:179-190. Transformations are accomplished as described by D. A. Morrison, *Methods Enzymol*. 68:326-331 (1979).

The transformed strains are grown overnight, e.g. about 16-18 hours, in LB/0.1% glucose with 100 »g/ml each L-tryptophan (trp) and ampicillin (AMP). For induction the cultures are transferred to M9/0.2 % glucose with 100 »g/ml AMP. Samples are collected for SDS PAGE, Western blots, and cell density (OD₅₅₀) determinations. Results indicate that LMS-73 displays an IGF expression phenotype with expression levels of about 100 times that of wild-type controls.

### Example 2 IGF Expression by LMS-117

### a) Generation of Tryptophan Repressor Mutant (trpR⁻) LMS-117

P1 phage transduction is used to further modify LMS-73. This modification consists of insertion of the marker gene coding for kanamycin resistance (kan) linked to a sequence coding for the protein cI⁸⁵⁷.

LMS-73 is grown to saturation in LB/0.1% glucose. 10 ml aliquots are centrifuged at 8000 x G for 10 minutes and the resulting pellet is resuspended in 5 ml 10mM magnesium sulfate and 5mM calcium chloride. 2.5 ml of phage P1^{(TAP106)} is added and transferred to a shaker flask (30° C, 200 rpm, 30 minutes). 5 ml 1M sodium citrate is added and cells pelleted (8000 x G, 15 minutes). The cell pellet is resuspended in 10 ml LB/0.1% glucose and 5 ml 1M sodium citrate and maintained at 30° C for 90 minutes. The cells are spread on large (22 x 22 cm) ABII NUNC agar plates supplemented with 12.5 »g/ml kan and maintained at 30° C overnight, e.g. 16-18 hours.

Subsequent to overnight incubation on the kanamycin selective agar, clones displaying kanamycin resistance are purified on ABII/Kan as putative *cI⁸⁵⁷* transductants. The presence of a functional cI⁸⁵⁷ repressor protein is confirmed by cross streaking clones against homo-immune and hetero-immune phage stocks at a permissive temperature (30° C) and a non-permissive temperature (42° C). All clones show temperature sensitivity and one clone, designated LMS-117, is saved for subsequent use.

### b) Construction of pRAL1

Plasmid pRAL1 is constructed as described here and as shown in Chart 1. This plasmid contains a P_{L} promoter, Trp2 ribosome binding site (Trp2 rbs) and the nucleotide sequence coding for IGF70. The Trp2 rbs and IGF70 are obtained via *HpaI/BamHI* digestion of pIGF70/Trp2. As can be seen in Chart 1, a *HpaI* site is located within the Trp promoter and upstream of the Trp rbs. In addition, a *BamHI* site is located downstream of the N-terminus of IGF70. Restriction enzyme digestion with *HpaI/BamHI* produces a 260 b.p. fragment containing Trp2 rbs adjacent to and upstream of IGF70 (Chart 1b).

Plasmid pWS50 (Chart 1c) is a pBR322 based vector containing a P_{L} promoter. This plasmid is described in Sisk, W.P., et al., (1986) *Gene* 48:183-193, which is incorporated by reference. pWS50 contains *NruI* and *BamHI* sites within the *cII* sequence downstream of P_{L}. After digestion a large (approx. 7 kb) fragment is recovered.

Restriction enzyme digestion is carried out according to supplier recommendations (New England Biolabs, Beverly MA) at 37° C for 90 minutes. Aliquots of the reaction mixture are loaded onto 1.5% TAE analytical agarose gel to verify the course of the reaction. The remaining reactants are placed on a preparative gel, poured, and run under similar conditions. Fluorescent bands are removed and purified using Gene Clean (BIO-101, LaJolla CA 92038). Purified fragments are separated on 1.5% TAE agarose gels.

The 7 Kb pWS50 fragment and the 260 b.p. pIGF70/Trp2 fragment are ligated overnight, e.g. 16-18 hours, at 15° C. The plasmids are transformed into TAP106, plated onto ABII/AMP, and grown overnight at 30° C. After overnight growth, 20 clones are selected and grown in 3 ml (each) LB/AMP at 30° C. Plasmid is prepared from the 3 ml cultures using alkaline-lysis mini-preparation (Sambrook, et al, *Molecular Cloning* (1989)) and subsequently digested with *ClaI* restriction endonuclease to verify the presence of an appropriate size restriction fragment. Seventeen of the 20 clones contain an insert of expected size. Three clones are selected for sequencing using an appropriate primer and a dideoxy sequencing kit (U.S. Biochemicals, Cleveland OH 44122) with ³²P-dATP. Sequence analysis verified the desired construction (Chart 1d). One clone is designated pRAL1.

### c) IGF Expression

LMS-117 (*trpR₅₅*, λ(*N:kan cI⁸⁵⁷*)) is transformed with pRAL1 following the procedure described above. After transformation eight colonies were picked onto ABII/AMP (100 »g/ml) and purified. A single colony is selected and grown overnight in LB/0.1% glucose/AMP (100 »g/ml) at 30° C. This culture is inoculated 1:100 in M9/1% casamino acids plus 100 »g/ml AMP and grown at 30° C to 0.5 OD₅₅₀. Induction occurs upon heat shift to 42° C. Results indicate that LMS-117 produces IGF-I in an amount that is about 100 times that of the control (TAP106).

### Example 3 IGF Expression by LMS-118

### a) Introduction of lon mutations into LMS-73

This modification consists of insertion of a mutant *lon* gene into LMS-73. LMS-73 is grown and treated with DU175C(106).1L as described in Example 2. In this case, the cells are plated onto Tet for selection. Clones are selected and verified for the presence of *lon*⁻. One such clone, designated LMS-79 (*trpR₅₅*, *lon::Tn10*), is saved for subsequent use. The clone is grown to saturation in LB/0.1% glucose supplemented with 15»g/ml Tet.

### b) Introduction of kan, cI⁸⁵⁷

The *lon*⁻ strain (LMS-79) is further modified utilizing TAP106 for insertion of the marker gene for kanamycin resistance (*kan*^{*r*}) linked to the *cI⁸⁵⁷* gene, as described in Example 2. Cells are treated, selected, and verified as described above. After transduction clones showing kanamycin resistance are purified, and verified as clones showing functional cI⁸⁵⁷ at 30° C and temperature sensitivity upon heat shift to 42° C. One such clone, designated LMS-118, is saved for subsequent use.

### c) IGF Expression

LMS-118 is transformed with pRAL1 as described above. Cells are purified and the presence of pRAL1 is verified as described above. Upon heat shift to 42° C, transformed LMS-118 expresses IGF-I in an amount that is about 100 times that of DU175C(106).1L, which serves as control.

### Example 4 Stability

### a) Pulse-chase experiments

Cultures are prepared from freshly transformed strains carrying the Trp2/IGF-I plasmid and grown to saturation overnight in LB at 37° C in the presence of L-tryptophan (100»g/ml) and ampicillin (100»g/ml). The IGF-I expression vector is induced by inoculating M9/0.1% CAA with a 1:100 dilution of the overnight culture. The M9 culture is grown to mid-log phase (0.6 OD₅₅₀) at 37° C, the bacterial cells are pelleted and resuspended in M9 defined media containing 17 amino acids minus tryptophan, methionine, and cysteine. The resuspended culture is grown at 37°C for 15 minutes, pulsed for 1 minute with 100 uCi/ml of culture with ³⁵S labelled methionine/cysteine and chased with 100 ug/ml of culture with unlabelled methionine and cysteine. One ml aliquots of the cultures are TCA precipitated at 0, 0.5, 1, 2.5, 5, 7.5, 10, 15, 30, and 60 minutes post-chase. The precipitated protein is pelleted, resuspended, and incubated with polyclonal anti IGF-I antibody using an antibody produced in-house. Suitable commercial preparations, e.g. from Research & Diagnostic Antibodies, Berkely CA or Temecon, Temecula CA, are also available and would give substantially the same result. The IgG component of the antibody/IGF-I complex is then bound to a Protein A-Sepharose matrix and the tertiary Protein A-antigen-antibody complex is pelleted, resuspended in DTT/SDS solution to separate the bound IGF-I, heat denatured, and loaded on a 15% separating gel.

### b) SDS PAGE and Radioanalytic Imaging

SDS PAGE is performed using the method of Laemmli (*Nature* 227:680-685 (1970)). Samples are loaded onto a 4% stacking gel and resolved on a 11.5 x 14 cm 15% separating gel for 3-4 hours at 40 milliamps/gel. ¹^{²}⁵I-IGF-I is loaded as a standard at concentration of 3,000 cpm to determine the appropriate immunoprecipitate bands. Following SDS/PAGE the gel proteins are fixed in 50% ethanol/10% glacial acetic acid for 2 hours followed by a one hour treatment in 15% ETOH/2.5% glycerol and dried at 60° C for 4 hours under vacuum. The dried gels are scanned with a radioisotope imaging system (Ambis Inc., San Diego CA) using a 0.40 x 3.2 mm screen for 10-24 hours and analyzed using commercial software (Ambis Inc., San Diego CA). Following the scan, the gels are placed in autoradiogram film cassettes and X-ray films exposed to confirm the radioanalytic image obtained from the computer analysis.

### c) Efficiency of Immunoprecipitation

The efficiency of the immunoprecipitation procedure is determined by spiking ¹²⁵I-IGF-I, as the antigen, at 1.45, 0.87, and 0.29 x 10⁵ cpm into 1 ml of post TCA buffer and taking this through the immunoprecipitation protocol.

### d) Results

Results of non-linear analysis for the strains assayed are listed in Table I. In summary, the half-life for the trpR⁻ mutants is longer then the half-life for the wild type strain which suggests an increased product stability in those strains.

Autoradiography of the dried gels (not shown) show the immunoprecipitate is co-migrating with ¹²⁵I-IGF standard indicating a monomeric IGF-I translation product is being quantified.

These results show that the tryptophan repressor mutants have an extended half-life over the wild-type strain.

The ability to measure the half-life of the expressed IGF-I under the defined conditions outlined in this work suggests that the increased expression seen with these strains is at least partially attributable to a greater stability of the IGF-I protein in these particular strains. In previous expression studies (results not shown) using both the *trpR₅₅* and *trpR₅₅ lon::Tn10* strains the Lon protease mutant did not show increased expression over the *trpR₅₅* strain suggesting that post-translational proteolysis by Lon protease is not a factor.

It is apparent that the foregoing description provides a means by which a host may be easily and efficiently altered to effect genomic changes therein and result in an organism with enhanced capacity for heterologous protein or peptide expression of highly stabile expression product.

**TABLE I**

| Strain | Genotype | Half-Life (min) |
|---|---|---|
| DU99B | wild-type | 5.7 |
| LMS-73 | *trpR₅₅* | 18.8 |
| LMS-79 | *trpR₅₅ lon::Tn10* | 10.6 |

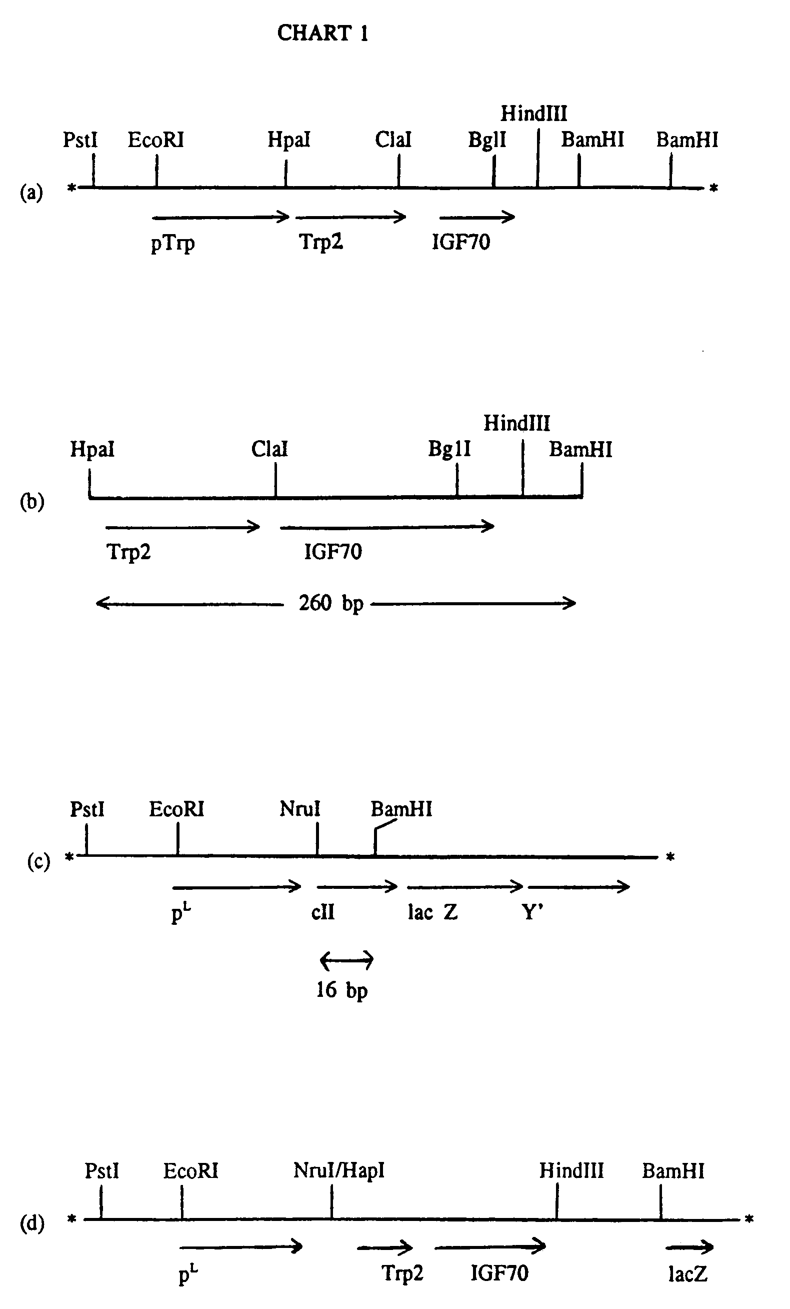
CHART 1

HindIII
PstI EcoRI HpaI ClaI BglI BamHI BamHI
(a) * *
pTrp Trp2 IGF70

HindIII
HpaI ClaI Bg1I BamHI
(b)
Trp2 IGF70
260 bp

PstI EcoRI NruI BamHI
(c) * *
p^L cII lac Z Y'
16 bp

PstI EcoRI NruI/HapI HindIII BamHI
(d) * *
p^L Trp2 IGF70 lacZ

## Claims

1. A host microorganism comprising a chromosomally-inserted non-functional TrpR55 gene, wherein the host is selected from E. coli strain LMS-73, having accession number NRRL B-18744, E. coli strain LMS-117, having accession number NRRL B-18763, and E. coli strain LMS-118, having accession number NRRL B-18764.

2. A host of claim 1, further comprising a vector encoding heterologous protein operably linked to a tryptophan promoter.

3. A host of claim 2, wherein the vector encodes porcine IGF-I.

4. A host of claim 3, wherein the vector is pIGF70/Trp2 or pRAL1, having accession number NRRL B-18779.

5. A host of claim 4, wherein the E. coli is LMS-73 and the vector is pIGF70/Trp2.

6. A host of claim 4, wherein the E. coli is LMS-117 and the vector is pRAL1.

7. A host of claim 4, wherein the E. coli is LMS-118 and the vector is pRAL1.

8. A method for enhancing the expression and stability of heterologous protein in E. coli, comprising culturing a host according to any of claims 2 to 7, and isolating the protein.

## Patentansprüche

1. Mikroorganismenwirt mit einem in das Chromosom insertierten nicht-funktionellen TrpR55-Gen, wobei der Wirt aus dem E. coli-Stamm LMS-73 mit der Zugangsnummer NRRL B-18744, dem E. coli-Stamm LMS-117 mit der Zugangsnummer NRRL B-18763 und dem E. coli-Stamm LMS-118 mit der Zugangsnummer NRRL B-18764 ausgewählt wird.

2. Wirt nach Anspruch 1, weiterhin umfassend einen für ein heterologes Protein, das in funktioneller Weise an einen Tryptophan-Promotor gebunden ist, kodierenden Vektor.

3. Wirt nach Anspruch 2, wobei der Vektor für Schweine-IGF-I kodiert.

4. Wirt nach Anspruch 3, wobei der Vektor pIGF70/Trp2 oder pRAL1 mit der Zugangsnummer NRRL B-18779 ist.

5. Wirt nach Anspruch 4, wobei das E. coli LMS-73 und der Vektor pIGF70/Trp2 ist.

6. Wirt nach Anspruch 4, wobei das E. coli LMS-117 und der Vektor pRAL1 ist.

7. Wirt nach Anspruch 4, wobei das E. coli LMS-118 und der Vektor pRAL1 ist.

8. Verfahren zur Verstärkung der Expression und der Stabilität eines heterologen Proteins in E. coli, umfassend das Kultivieren eines Wirts nach einem der Ansprüche 2 bis 7 und Isolieren des Proteins.

## Revendications

1. Micro-organisme servant d'hôte, comprenant un gène TrpR₅₅ non fonctionnel inséré dans le chromosome, l'hôte étant choisi entre la souche E. coli LMS-73, portant le numéro de dépôt NRRL B-18744, la souche E. coli LMS-117, portant le numéro de dépôt NRRL B-18763 et la souche E. coli LMS-118, portant le numéro de dépôt NRRL B-18764.

2. Hôte suivant la revendication 1, comprenant en outre un vecteur codant pour une protéine hétérologue, lié de manière fonctionnelle à un promoteur tryptophane.

3. Hôte suivant la revendication 2, dans lequel le vecteur code pour le IGF-I porcin.

4. Hôte suivant la revendication 3, dans lequel le vecteur est pIGF70/Trp2 ou pRAL1, portant le numéro de dépôt NRRL B-18779.

5. Hôte suivant la revendication 4, dans lequel la souche de E. coli est LMS-73 et le vecteur est pIGF70/Trp2.

6. Hôte suivant la revendication 4, dans lequel la souche de E. coli est LMS-117 et le vecteur est pRAL1.

7. Hôte suivant la revendication 4, dans lequel la souche de E. coli est LMS-118 et le vecteur est pRAL1.

8. Procédé pour accroître l'expression et la stabilité d'une protéine hétérologue dans E. coli, comprenant la culture d'un hôte suivant l'une quelconque des revendications 2 à 7, et l'isolement de la protéine.
